# EUROPEAN PATENT APPLICATION

(11) **EP 1 072 224 A2**
(43) Date of publication of application: **31.01.2001**
(21) Application number: 00306245.2
(22) Date of filing: 21.07.2000
(51) Int. Cl.: A61B 6/03

(54) **Retrospective cardiac gating with cine scans on a multislice scanner**

(30) Priority: 28.07.1999 US 362511
(71) Applicant: GENERAL ELECTRIC COMPANY, Schenectady, NY 12345 (US)
(72) Inventor: Woodford, Mark, Waukesha, Wisconsin 53188 (US); Acharya, Kishore C., Brookfield, Wisconsin 53005 (US); Fox, Stanley H., Brookfield, Wisconsin 53045 (US); Pan, Tin Su, Brookfield, Wisconsin 53005 (US)
(74) Representative: Pedder, James Cuthbert

(57) **Abstract**

A method for obtaining a set of still image slices of a moving heart of a patient (22) utilizes a multi-slice CT scanner (10) and an EKG data recorder (52). The method includes steps of simultaneously recording EKG data (60) of the patient's cardiac utilizing the EKG data recorder while cine scanning and recording plural CT image slices (62, 66, 70) of the patient's heart utilizing the multi-slice CT scanner; correlating the recorded cine scanned CT image slices with the recorded EKG data; and selecting segmented images (64, 68, 72) of the set of recorded image slices corresponding to a selected portion of the patient's cardiac cycle as indicated by the correlated, recorded EKG data, the selected segmented images thereby becoming the set of still image slices.

## Description

This invention relates generally to methods for noninvasive diagnostic imaging of a patient, and more particularly to methods for collecting still images of a patient's moving heart utilizing a CT scanning device.

In one known diagnostic evaluation, a physician screens otherwise healthy patients for cardiac lesions and plaque by examining and scoring x-ray images of the patient's heart. For example, calcium-containing plaque can be readily observed in such images and risk levels associated with an observed amount of plaque estimated by a trained physician. However, for proper and complete evaluation, a sufficient number of still image slices must be obtained to provide adequate coverage of the heart. One known method for performing cardiac evaluations employs a computed tomography (CT) scanning imaging system to obtain slices having the needed coverage.

In at least one known CT imaging system configuration, an x-ray source projects a fan-shaped beam which is collimated to lie within an X-Y plane of a Cartesian coordinate system and generally referred to as the "imaging plane". The x-ray beam passes through the object being imaged, such as a patient. The beam, after being attenuated by the object, impinges upon an array of radiation detectors. The intensity of the attenuated beam radiation received at the detector array is dependent upon the attenuation of the x-ray beam by the object. Each detector element of the array produces a separate electrical signal that is a measurement of the beam attenuation at the detector location. The attenuation measurements from all the detectors are acquired separately to produce a transmission profile.

In known third generation CT systems, the x-ray source and the detector array are rotated with a gantry within the imaging plane and around the object to be imaged so that the angle at which the x-ray beam intersects the object constantly changes. A group of x-ray attenuation measurements, i.e., projection data, from the detector array at one gantry angle is referred to as a "view". A "scan" of the object comprises a set of views made at different gantry angles, or view angles, during one revolution of the x-ray source and detector. In an axial scan, the projection data is processed to construct an image that corresponds to a two-dimensional slice taken through the object. One method for reconstructing an image from a set of projection data is referred to in the art as the filtered back projection technique. This process converts the attenuation measurements from a scan into integers called "CT numbers" or "Hounsfield units", which are used to control the brightness of a corresponding pixel on a cathode ray tube display. In one known method for obtaining still images of a patient's heart for calcium scoring, a CT scanner is employed in a spiral scanning mode. A patient being evaluated remains still on a table of the CT scanner as the table continuously advances through the scanner along a z-axis, i.e., along the axis of rotation of the gantry. The patient holds his or her breath while the scan is taken to ensure that, with the exception of the heart, no other part of the body is moving. With one known scanner, this method requires about 40 seconds to complete and results in a collection of 400 images out of which 40 are selected manually for scoring. However, because there is no coordination between the movement of the table and the beating of the patient's heart, it is not possible to ensure that the best views for scoring provide full coverage of the heart. As a result, even the best manually selected views may not provide sufficient coverage of the heart for proper scoring. One way to overcome this difficulty is to advance the table of the CT scanner more slowly so that, for a given z-axis advancement (i.e., advancement of the table in a direction along its length), the scanner obtains more images. This modification, however, requires a patient to hold his or her breath a proportionately longer time to complete the scanning. For patients in average health, however, holding one's breath for longer than 40 seconds is at least uncomfortable, and may be impossible.

In another known method for providing CT scanned images of a heart suitable for diagnostic scoring, a patient is hooked to an EKG device and the scanner is synchronized to a signal derived from the patient's EKG. This method represents an improvement over the preceding method, because it provides a level of control over, or at least knowledge of a z-axis location of an imaging plane relative to the patient's heart during specified portions of a cardiac cycle. However, synchronizing the motions of the table and the rotating gantry to a patient's heartbeat increases the complexity of a CT scanner, even when the patient's heartbeat is relatively constant. Of course, most patients are unfamiliar with the testing environment and understandably become nervous during the test. This nervousness introduces a level of variability into the patient's heartbeat on a time scale can impact the test procedure.

In another known method for providing cardiac images suitable for scoring, an electron beam is scanned across a curved metal plate to produce x-ray emissions that are directed towards the patient. Because only an electron beam is scanned, there is no need for a rotating gantry and the heart can be scanned very rapidly. However, at present, electron-beam scanners are rare and expensive in comparison to CT scanners.

It would therefore be desirable to provide a method to collect a complete set of still images of a moving heart utilizing standard, relatively inexpensive equipment. It would also be desirable to if such a method provided a suitable set of still images from predictable *z*-axis positions without requiring a patient to hold his or her breath for an excessive period of time.

According to a first aspect of the invention, there is provided a method for obtaining a set of still images of a moving heart of a patient utilizing a multi-slice CT scanner and an EKG data recorder, said method comprising: simultaneously recording EKG data of the patient's cardiac cycle utilizing the EKG data recorder while cine scanning and recording plural CT image slices of the patient's heart utilizing the multi-slice CT scanner; correlating the recorded cine scanned CT image slices with the recorded EKG data; and selecting segmented images of the set of recorded image slices corresponding to a selected portion of the patient's cardiac cycle as indicated by the correlated, recorded EKG data, the selected segmented images thereby becoming the set of still image slices.

The selected portion of the cardiac cycle may is preferably a portion in which the heart is relatively stationary.

The CT scanner may have a rotating gantry, a detector, and an x-ray source on the rotating gantry projecting an x-ray beam at a detector, and the detector may acquire a fan angle of data for each image slice, and wherein cine scanning plural CT image slices of the patient's heart may comprise the step of collecting projection data from an axial scan having a duration at least as long as one complete cardiac cycle plus a time required for rotation of the gantry through 180° plus an angle equal to or less than one fan angle.

The selected portion of the patient's cardiac cycle may be a portion of the cardiac cycle immediately prior to R-peaks of the recorded EKG data.

Selecting segmented images of the set of recorded image slices may comprise the step of selecting segments of the set of collected image slices ending immediately prior to the EKG R-peaks or may comprise the step of selecting segmented images collected during rotation of the gantry through at least 180° plus an angle equal to or less than one fan angle.

The multi-slice CT scanner may comprise a moveable table on which the patient is resting, and the method may further comprise stepping the table so that EKG data and CT image slices are recorded simultaneously at the plurality of positions.

The selected portion of the patient's cardiac cycle may be a portion of the cardiac cycle in which the heart is relatively stationary.

The CT scanner may have a rotating gantry, a detector, and an x-ray source on the rotating gantry projecting an x-ray beam at a detector, and the detector may acquire a fan angle of data for each image slice, and wherein cine scanning plural CT image slices of the patient's heart may comprise the step of collecting projection data from an axial scan having a duration at least as long as one complete cardiac cycle plus a time required for rotation of the gantry through 180° plus an angle equal to or less than one fan angle.

The selected portion of the patient's cardiac cycle may be a portion of the cardiac cycle immediately prior to R-peaks of the recorded EKG data.

Selecting segmented images of the set of recorded image slices may comprises the step of selecting segments of the set of collected image slices ending immediately prior to the EKG R-peaks or may comprise the step of selecting segmented images collected during rotation of the gantry through at least 180° plus one fan angle.

Cine scanning and recording plural CT image slices of the patient's heart may comprise simultaneously cine scanning and acquiring data for at least four image slices.

Cine scanning and recording data for at least four image slices may comprise acquiring data for at least four images slices each having a thickness of between 2.5 and 3.75 mm.

Stepping the table may comprise stepping the table a distance of a total of the thicknesses of the simultaneously acquired image slices.

Selecting segments of the set of collected image slices may comprise selected segments having at least 120 mm coverage of the patient's heart, and simultaneously recording EKG data of the patient's cardiac cycles while scanning and recording plural CT image slices of the patient's heart may comprise scanning for a total of no more than 30 seconds.

According to a second aspect of the invention, there is provided a system for obtaining a set of still image slices of a moving heart of a patient, said apparatus configured to: simultaneously record EKG data of a patient's cardiac cycle while cine scanning and recording plural CT image slices of the patient's heart; and correlate a recording time of the recorded EKG data with the recorded plural CT image slices.

The system may comprise an EKG apparatus configured to record EKG data and to record, in association with the recorded EKG data, an indication of a start of a simultaneous recording of CT image slices.

The system may further comprise a display screen configured to display a CT image slice from the recording of CT image slices and a portion of the simultaneously recorded EKG data, including an indication of a portion of the EKG data corresponding to a time of recording of the CT image slice being displayed.

The system may comprise a multi-slice CT scanner configured to simultaneously record at least four image slices each having a thickness of between 2.5 and 3.75 mm.

According to a third aspect of the invention, there is provided a system for retrospectively correlating recorded CT images and recorded EKG data, said system being configured to record EKG data of a patient's cardiac cycle; and said system being configured to be responsive to a CT image scanner to record an indication correlating a start of a CT scan with a simultaneously recorded portion of recorded EKG data.

The system may be configured to record the EKG data and the indication correlating a start of the CT scan with a simultaneously recorded portion of the recorded EKG data on a floppy disk.

Thus there is therefore provided, in one embodiment, a method for obtaining a set of still image slices of a moving heart of a patient utilizing a multi-slice CT scanner and an EKG data recorder. The method includes steps of simultaneously recording EKG data of the patient's cardiac utilizing the EKG data recorder while cine scanning and recording plural CT image slices of the patient's heart utilizing the multi-slice CT scanner; correlating the recorded cine scanned CT image slices with the recorded EKG data; and selecting segmented images of the set of recorded image slices corresponding to a selected portion of the patient's cardiac cycle as indicated by the correlated, recorded EKG data, the selected segmented images thereby becoming the set of still image slices. The selected segments thus become the set of still image slices. This method embodiment does not require that the CT scanner be synchronized to the EKG of the patient, because the acquired EKG data and scanned image data can be synchronized after they are recorded. Moreover, no special CT scanning technology, such as electron-beam scanning, is required, yet a suitable set of still images of the patient's heart can be acquired in a period of time in which a normal patient is able to hold his or her breath.

The invention will now be described in greater detail, by way of example, with reference to the drawings, in which:-
Figure 1 is a pictorial view of a CT imaging system and EKG recording device.
Figure 2 is a block schematic diagram of the system and EKG recording device illustrated in Figure 1.
Figure 3 is a diagram illustrating a gantry rotation requirement for obtaining a segmented image.
Figure 4 is a diagram illustrating a relationship between CT x-ray exposures, segmented images selected from the CT x-ray exposures and an EKG recording.
Figure 5 is a drawing of a workstation display facilitating selection of segmented still images for study.

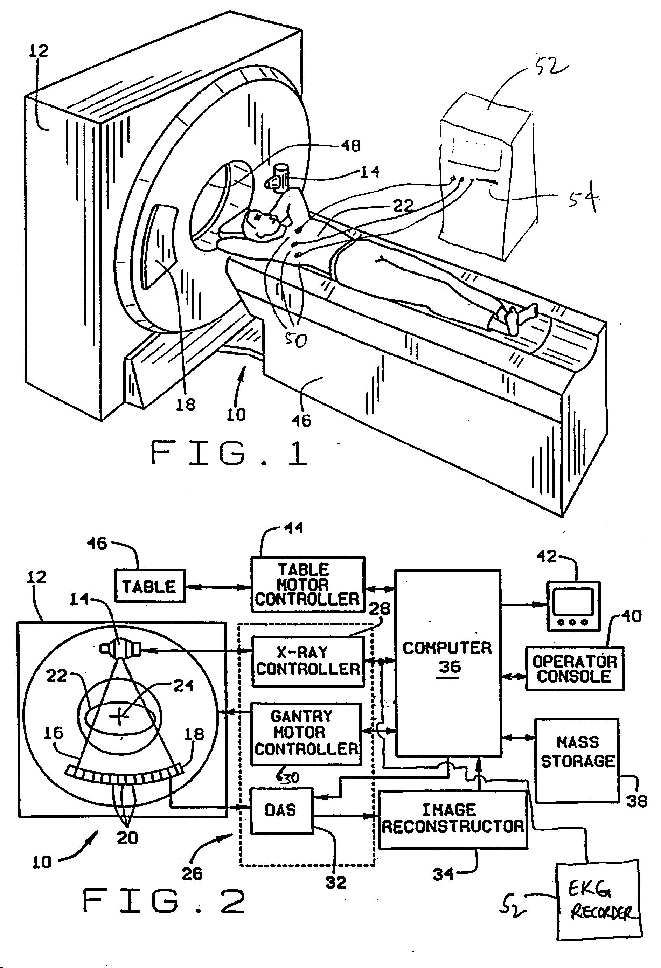

Referring to Figures 1 and 2, a computed tomograph (CT) imaging system 10 is shown as including a gantry 12 representative of a "third generation" CT scanner. Gantry 12 has an x-ray source 14 that projects a beam of x-rays 16 toward a detector array 18 on the opposite side of gantry 12. Detector array 18 is formed by detector elements 20, which together sense the projected x-rays that pass through an object 22, for example a medical patient. Detector array 18 is fabricated in a multi-slice configuration. This type of configuration is found in at least one known commercial imaging system. Each detector element 20 produces an electrical signal that represents the intensity of an impinging x-ray beam and hence the attenuation of the beam as it passes through patient 22. During a scan to acquire x-ray projection data, gantry 12 and the components mounted thereon rotate about a center of rotation 24.

Rotation of gantry 12 and the operation of x-ray source 14 are governed by a control mechanism 26 of CT system 10. Control mechanism 26 includes an x-ray controller 28 that provides power and timing signals to x-ray source 14 and a gantry motor controller 30 that controls the rotational speed and position of gantry 12. A data acquisition system (DAS) 32 in control mechanism 26 samples analog data from detector elements 20 and converts the data to digital signals for subsequent processing. An image reconstructor 34 receives sampled and digitized x-ray data from DAS 32 and performs high speed image reconstruction. The reconstructed image is applied as an input to a computer 36 that stores the image in a mass storage device 38.

Computer 36 also receives commands and scanning parameters from an operator via console 40 that has a keyboard. An associated cathode ray tube display 42 allows the operator to observe the reconstructed image and other data from computer 36. The operator supplied commands and parameters are used by computer 36 to provide control signals and information to DAS 32, x-ray controller 28 and gantry motor controller 30. In addition, computer 36 operates a table motor controller 44 that controls a motorized table 46 to position patient 22 in gantry 12. Particularly, table 46 moves portions of patient 22 through gantry opening 48.

In one embodiment, to obtain a set of still image slices of a moving heart of patient 22, EKG data of the cardiac cycles of patient 22 is recorded while plural CT image slices of the patient 22 heart are scanned and recorded with a multi-slice CT scanner. EKG data is obtained by connecting EKG probes 50 from an EKG recorder 52 to patient 22. EKG recorder 52 comprises a floppy disk drive 54 that is utilized for recording EKG data from patient 22 on a diskette. EKG recorder 52 also monitors a signal from CT imaging system 10 indicating a start of a scan, for example, a signal from computer 36 instructing x-ray controller 28 to energize x-ray source 14. An indication of the occurrence of this signal is recorded on the diskette in floppy disk drive 54 with the EKG data to indicate a time at which the EKG data coincides with the start of a scan. Recording a start of scan signal in this manner permits later correlation of the EKG data taken by EKG recorder 52 and image data obtained by CT imaging system 10 after completion of a recording. As used herein, "recording plural CT image slices" is intended to encompass recording of data representative of plural CT image slices.

In at least one knows commercial embodiment of a multi-slice scanner, helical scan capability is provided so that table 46 continuously moves portions of patient 22 through gantry opening 48, i.e., in a *z*-direction, as image data is being collected. However, in one embodiment of the present invention, imaging system 10 provides cine rather than helical scans of patient 22, i.e., table 46 is held motionless while axial scanning is performed and image data is continuously recorded. Imaging system 10 is therefore configured to provide such scans, for example, by programming computer 36 of imaging system 10 to send appropriate signals to table motor controller 44, x-ray controller 28, and gantry motor controller 30 to carry out a cine scanning function upon entry of a command at operator console 40 after patient 22 and table 46 are positioned for scanning with x-ray source 14 and detector array 18.

Multiple image slices are recorded simultaneously by scanner 10. For example, one known multi-slice scanner has 16 detector rows that are combinable to provide image slices having thicknesses of 1.25 mm or multiples thereof. This scanner also has the capability to simultaneously record up to 4 image slices at the same time. Image slice thicknesses of approximately 3.0 mm such as those that are desirable for calcification scoring can be approximated with the detector 18 and DAS 32 configuration of such a scanner by combining detector rows in thicknesses of either 2.5 or 3.75 mm. Configurations in which more than 4 slices can obtained simultaneously are possible with suitable detector 18 and DAS 32 hardware, and such configurations can be advantageously employed in practicing the present invention.

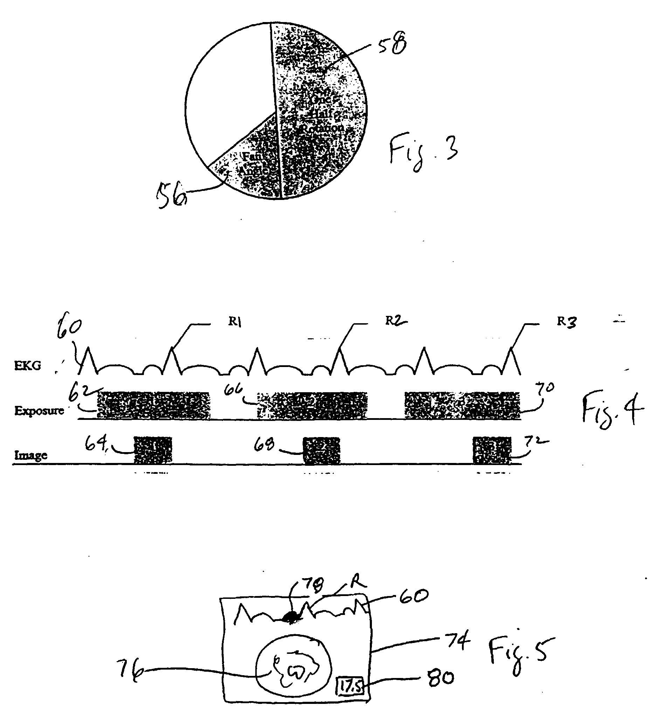

In one embodiment of the invention, the still images of a patient's heart that are obtained are "segmented images." These are images made in the shortest period of time needed to make an image with a mechanical CT scanner. Referring to Figure 3, a segmented image is computed from data obtained during a half rotation 58 (i.e., 180°) of gantry 12 plus an additional amount equal to or less than one fan angle 56, which is the detector width angle. In one embodiment, for example, gantry 12 rotates once every 0.8 seconds and through a fan angle 56 in about 0.13 seconds. Thus, a segmented image in this embodiment corresponds to 0.53 seconds of continuous data. It will be understood that better images are obtained with faster gantry rotation rates, which make shorter scanning periods possible.

Optimum segmented images for calcification scoring are those obtained during the most stationary period of the cardiac cycle. This period is selected by noting that the human heart demonstrates minimum motion from the time it is almost full of blood until the systole, which is when the myocardium contracts quickly to pump blood out. To ensure collection of image data of the heart during its most stationary period, scanning system 10 collects cine image data for a time duration at least equal to the time required for rotation of gantry 12 through one fan angle 56 plus one half rotation 58, plus one complete cardiac cycle. A segmented image is created using data from the segment corresponding to the most stationary portion of the cardiac cycle.

To select images corresponding to the most stable period of the cardiac cycle, the floppy disk recorded by EKG recorder 52 and reconstructed image data from mass storage 38 and computer 36 are both loaded into a computer workstation (not shown). Referring to Figure 4, with the aid of the start of scan signal recorded on the floppy disk, an EKG data signal 60 is correlated with image data 62. A large spike R1 in EKG data signal 60 corresponds to an R-peak, which is an indication that systole is imminent. A segment 64 ending very close to an EKG R-peak is optimal for creating an image with a minimum of motion artifacts. Because a plurality of image slices are collected by CT imaging system 10 simultaneously, selecting a set of segmented image slices 64 taken at the optimum time produces the best images for scoring.

When four slices at a time are obtained by CT imaging system 10, thirty-two or more segmented image slices providing full heart coverage can be readily obtained without requiring that patient 22 hold his or her breath for an excessive period of time. For example, at least one known multi-slice scanner 10 has hardware capable of rotating gantry 12 a full 360° in 0.8 seconds. A cine scan of two full rotations (1.6 seconds) at a first location of patient 46 suffices to provide four slices of the patient's heart because the time required for two full rotations is greater than a half rotation 58 plus a fan angle 56, plus one complete cardiac cycle. (A longer scan may be required if the cardiac cycle is unusually slow.) Thus, in one embodiment, table 46 is stepped to another position by computer 36 and table motor controller 44 between cine scans of 1.6 second duration. Each step advances table 46 a distance four times the slice width, so that each cine scan (other than the first) provides image slices contiguous with, but not overlapping the image slices obtained during the cine scan immediately preceding the most recent table 46 step. (It will be understood that the stepping distance is adjusted in accordance with the number of slices recorded simultaneously during a cine scan.) Each table step can be accomplished in 1.5 seconds with known motorized tables 46 and controllers 44. Therefore, the total time required to obtain 32 segmented images while patient 22 holds his or her breath is 0.8 seconds per rotation per scan x 2 rotations x 8 scans + 7 table steps between scans x 1.5 seconds per table step = 23.3 seconds. Most patients 22 to be screened for calcification would be able to hold their breath this length of time without much difficulty. With 3.75 mm segmented image slices, a total of 120 mm of coverage is obtained in this amount of time, a coverage sufficient for calcification scoring. If patient 22 can hold his or her breath for a longer period, more slices can be obtained from a four-slice system 10. For example, 30 seconds is sufficient to obtain 40 segmented images. It will be understood that the total amount of coverage obtainable is dependent upon the number of slices available per cine scan, the width of the slices, and the amount of time patient 22 can hold his or her breath.

Each cine scan in the scan-step-scan-step procedure produces a set of image data. By way of illustration, three such sets of image data 62, 66, and 70 are indicated in Figure 4. Sets of segmented image slices 64, 68, and 72 are selected for the still images of the heart at portions of each set of image data 62, 66, and 70. Segmented image slices 64, 68, and 72 each immediately precede R-peaks R1, R2, and R3 in Figure 4. Those skilled in the art will understand that although exposures 62, 66, and 70 are shown as being discontinuous in Figure 4, it is not necessary to turn off the x-ray beam or stop collecting image data during steps of table 46. However, if continuous image data is obtained from scanner 10, it is useful for the recorded image data to include indications distinguishing data taken during cine scans and data taken while table 46 is stepping.

Figure 5 is a drawing of an embodiment of a workstation display 74 that facilitates manual selection of image data segments 64, 68, and 72 from exposures 62, 66, and 70. A portion of EKG data signal 60 is shown above a reconstructed, segmented image 76. Operator controls, such as a mouse or keyboard (neither of which are shown), are operated to step segmented image 76 in time through the recorded scanned image data. As segmented image 76 is stepped in time, EKG data signal 60 is scrolled, and a portion 78 of EKG data signal 60 that corresponds to a presently displayed segmented image 76 appears highlighted on display 74. By observing highlighted portion 78, segmented images 76 correlated with a selected portion R of a cardiac cycle are located, and corresponding images from each slice taken by multi-slice scanner 10 are selected at the same time. By stepping segmented image 76 through the entire set of image data and selecting cine scanned data at different positions at corresponding portions of the cardiac cycle, a set of segmented still images are selected for scoring. To facilitate selection, a table *z*-location 80 or another suitable indication of scanning plane location is recorded along with the image data and displayed on workstation screen 74 with each segmented image 76.

From the preceding description of various embodiments of the present invention, it is evident that a complete set of still images of a moving heart are obtained at predictable *z*-axis positions using relatively inexpensive equipment. Moreover, the still images are obtained without requiring a patient to hold his or her breath for an excessive period of time.

Although the invention has been described and illustrated in detail in connection with specific embodiments, it is to be clearly understood that the same is intended by way of illustration and example only and is not to be taken by way of limitation. For example, data from EKG recorder 52 need not be recorded on a floppy disk. In another embodiment, for example, data is transferred from EKG recorder 52 to a workstation either via a serial port or via a network connection. In another embodiment, computer 36 is programmed to provide the workstation functions for selecting segmented image data 64, 68, and 72, using operator console 40 and display screen 42. It will also be observed that a trade-off exists at any given gantry rotation speed between an amount of time a patient 22 may have to hold his or her breath and a number of segmented still image slices required for diagnostic purposes. A multi-slice scanner 10 equipped to simultaneously acquire a greater number of slices than those of the exemplary embodiments would require less time to acquire a set of images with sufficient coverage for diagnostic calcification scoring at the same gantry rotation speed. Faster gantry rotation speeds would also reduce image acquisition time with suitable x-ray sources and detectors. In addition, the CT system described herein is a "third generation" system in which both the x-ray source and detector rotate with the gantry. Many other CT systems including "fourth generation" systems wherein the detector is a full-ring stationary detector and only the x-ray source rotates with the gantry, may be used if individual detector elements are corrected to provide substantially uniform responses to a given x-ray beam.

## Claims

1. A method for obtaining a set of still image slices (76) of a moving heart of a patient (22) utilizing a multi-slice CT scanner (10) and an EKG data recorder (52), said method comprising:
simultaneously recording EKG data (60) of the patient's cardiac cycle utilizing the EKG data recorder while cine scanning and recording plural CT image slices (62, 66, 70) of the patient's heart utilizing the multi-slice CT scanner;
correlating the recorded cine scanned CT image slices with the recorded EKG data; and
selecting segmented images (64, 68, 72) of the set of recorded image slices corresponding to a selected portion (78) of the patient's cardiac cycle as indicated by the correlated, recorded EKG data, the selected segmented images thereby becoming the set of still image slices.

2. A method in accordance with Claim 1 wherein the selected portion (78) of the cardiac cycle is a portion in which the heart is relatively stationary.

3. A method in accordance with Claim 1 or 2 wherein the CT scanner (10) has a rotating gantry (12), a detector (18), and an x-ray source (14) on the rotating gantry projecting an x-ray beam at a detector, and the detector acquires a fan angle (56) of data for each image slice, and wherein cine scanning plural CT image slices (62, 66, 70) of the patient's heart comprises the step of collecting projection data from an axial scan having a duration at least as long as one complete cardiac cycle plus a time required for rotation of the gantry through 180° (58) plus an angle equal to or less than one fan angle.

4. A method in accordance with Claim 3 wherein the selected portion (78) of the patient's cardiac cycle is a portion of the cardiac cycle immediately prior to R-peaks of the recorded EKG data (60).

5. A system (10) for obtaining a set of still image slices (76) of a moving heart of a patient (22), said apparatus configured to:
simultaneously record EKG data (60) of a patient's cardiac cycle while cine scanning and recording plural CT image slices (62, 66, 70) of the patient's heart; and
correlate a recording time of the recorded EKG data with the recorded plural CT image slices.

6. A system in accordance with Claim 5 wherein said system comprises an EKG apparatus (52) configured to record EKG data (60) and to record, in association with the recorded EKG data, an indication of a start of a simultaneous recording of CT image slices (62, 66, 70).

7. A system (10) in accordance with Claim 6 and further comprising a display screen (74) configured to display a CT image slice from the recording of CT image slices (62, 66, 70) and a portion of the simultaneously recorded EKG data (60), including an indication of a portion of the EKG data corresponding to a time of recording of the CT image slice being displayed.

8. A system (10) in accordance with Claim 7 wherein said system comprises a multi-slice CT scanner configured to record simultaneously at least four image slices (62, 66, 70) each having a thickness of between 2.5 and 3.75 mm.

9. A system (10) for retrospectively correlating recorded CT images (62, 66, 70) and recorded EKG data (60), said system being configured to record EKG data of a patient's cardiac cycle; and said system being configured to be responsive to a CT image scanner to record an indication correlating a start of a CT scan with a simultaneously recorded portion of recorded EKG data.

10. A system (10) in accordance with Claim 9 configured to record the EKG data (60) and the indication correlating a start of the CT scan with a simultaneously recorded portion of the recorded EKG data on a floppy disk.
